# EUROPÄISCHE PATENTSCHRIFT

(11) **EP 2 614 771 B1**
(45) Veröffentlichungstag und Bekanntmachung des Hinweises auf die Patenterteilung: **10.08.2016**
(21) Anmeldenummer: 13150340.1
(22) Anmeldetag: 07.01.2013
(51) Int. Cl.: A61B 5/00, A61B 5/053, A61B 5/024, A61B 5/08, A61B 5/16

(54) **Verfahren zur Bioimpedanzmessung**
Method for measuring bio-impedance
Procédé destiné à mesurer la bioimpédance

(30) Priorität: 13.01.2012 DE 102012100295
(43) Veröffentlichungstag der Anmeldung: 17.07.2013
(73) Patentinhaber: movisens GmbH, 76133 Karlsruhe (DE); Karlsruher Institut für Technologie, 76131 Karlsruhe (DE)
(72) Erfinder: Ottenbacher, Jörg, 76135 Karlsruhe (DE); Großmann, Ulrich, 76344 Eggenstein (DE); Stumpp, Jürgen, 76337 Waldbronn (DE); Hey, Stefan, 76831 Impflingen (DE); Stork, Wilhelm, 76829 Landau (DE)
(74) Vertreter: Geitz Truckenmüller Lucht

(56) Entgegenhaltungen:
- EP-A1- 2 158 838
- EP-A1- 2 364 643
- WO-A1-00/28892
- WO-A1-2004/112606
- WO-A2-2008/094077
- FR-A1- 2 912 049
- US-A- 5 823 957
- US-A1- 2008 119 329
- US-A1- 2010 280 394
- US-B1- 7 628 757
- US-B1- 7 925 349

## Beschreibung

Die vorliegende Erfindung betrifft ein Verfahren zur Erfassung von Vitalparametern mithilfe einer am Handgelenk tragbaren Bioimpedanz-Messvorrichtung, wobei mithilfe wenigstens eines ersten Elektrodensatzes, bestehend aus einem Paar von Einspeiseelektroden zur Einleitung eines Messstroms in die Haut eines Benutzers und einem zwischen den Einspeiseelektroden voneinander beabstandet angeordneten Paar von Messelektroden zur Erfassung des Messstroms über die Haut, ein Bioimpedanzsignal erfasst wird, aus welchem durch Tiefpassfilterung ein Atmungssignal und durch Hochpassfilterung ein Blutpulssignal abgeleitet werden und das Atmungs- und das Blutpulssignal einer Signalauswertung zur Bestimmung abgeleiteter Parameter zugeführt werden, sowie eine Bioimpedanz-Messvorrichtung umfassend ein am Handgelenk tragbares Trägerband, an welchem ein erster Elektrodensatz zur Erfassung eines Bioimpedanzsignals unter Kontaktierung der Haut eines Benutzers angeordnet ist, welcher aus einem Paar von Einspeiseelektroden zur Einleitung eines Messstroms in die Haut und einem zwischen den Einspeiseelektroden voneinander beabstandet angeordneten Paar von Messelektroden zur Erfassung der Spannung über die Haut besteht.

Ein derartiges Verfahren und auch eine solche Vorrichtung sind bereits aus der internationalen Patentanmeldung WO 00/28892 A1 vorbekannt. Gegenstand dieser internationalen Patentanmeldung ist eine Überwachungsvorrichtung, welche am Handgelenk eines Benutzers befestigt werden kann, wobei einem Gelenkband ein Elektrodensatz, bestehend aus zwei Einspeiseelektroden und zwei dazwischen angeordneten Messelektroden, zugeordnet ist. Mithilfe der Einspeiseelektroden wird ein Stromimpuls in die Haut eingespeist, dessen Effekt mithilfe der Messelektroden nachvollzogen wird, um hieraus Rückschlüsse auf die Impedanz der Haut ziehen zu können. Im Rahmen einer Bioimpedanzplethysmographie werden dann Erkenntnisse über Volumenänderungen der Gefäße gesammelt und von diesen Volumenänderungen auf Atmungs- und Pulsraten zurückgeschlossen.

Weitere Verfahren und Vorrichtungen dieser und ähnlicher Art sind aus den Schriften US 2008/0119329 A1 und FR 2 912 049 A1 bekannt.

Im Rahmen derartiger Messungen, welche üblicherweise aufgrund einer längeren Überwachung des Benutzers auch mobil sein sollten, treten Probleme aufgrund von Signalverzerrungen auf, wobei ein solches Verzerrungsereignis als "Artefakt" bezeichnet wird. Beispielsweise im Rahmen von Körperbewegungen, welche zum einen das Armband betreffen, zum anderen aber auch innerhalb des Körpers stattfinden, können die Messungen beeinflusst werden, so dass in der Messung ungeeignete Werte auftreten können. Beispielsweise werden beim Gehen im Rahmen jedes Schrittes Stöße durch den Körper weitergeleitet, welche sich auch in der Bioimpedanz niederschlagen. Nachdem einem Armband wie in der oben genannten WO 00/28892 A1 keine Möglichkeit zur Verfügung steht, festzustellen, ob sich eine Person in Bewegung oder in Ruhe befindet, wird das entsprechende Gerät auch lediglich in der Lage sein, die gemessenen Werte unvoreingenommen als gegeben zu betrachten und nicht zu hinterfragen.

Einen Schritt weiter geht diesbezüglich die deutsche Offenlegungsschrift DE 10 2005 059 435 A1, welche eine Vorrichtung zur nichtinvasiven Blutdruckmessung betrifft. Diese Vorrichtung umfasst einen Beschleunigungssensor, welcher eine Bewegung des auch hier vorgesehenen Armbandes in den Raumrichtungen erfassen kann und somit in die Lage versetzt wird, eine Bewegung des Benutzers zu erkennen und zu berücksichtigen. So wird bei einer starken Bewegung des Armbandes in eine Raumrichtung davon ausgegangen werden können, dass der Benutzer des Armbandes sich gerade fortbewegt, insbesondere geht, läuft oder zumindest aufgrund der Armbewegung, auf die der Beschleunigungssensor reagiert, in körperlicher Bewegung ist. Im Falle einer Kopplung des Beschleunigungssensors mit einem plethysmographischen Verfahren wird jedoch gelehrt, dass hierfür ein EKG-Messgerät erforderlich ist, welches zumindest einen zusätzlichen Aufwand bedeutet, üblicherweise sogar der Gesamtanordnung die Mobilität nehmen kann. Die dort gelehrte Möglichkeit zur Blutdruckmessung ist daher nur unter Einschränkungen realisierbar.

Sämtlichen dieser Vorrichtungen ist also gemein, dass sie ein möglichst vollständiges und verlässliches Bild der durchgeführten Messungen entweder nur in Ruhe oder unter den genannten Einschränkungen ermöglichen, und ferner auch nur in der Lage sind, dem Benutzer relativ rohe Daten zu liefern. Eine Gesamtbetrachtung der körperlichen Situation ergibt sich für den Benutzer aus diesen Daten erst durch eigene Kenntnis zur Auswertung, welche mit einigem zusätzlichem Aufwand erworben und angewendet werden müssten.

Vor diesem Hintergrund ergibt sich ein Themenkomplex, welcher der vorliegenden Erfindung als Aufgabe zu Grunde liegt. Es ist Aufgabe der Erfindung, einerseits ein Verfahren vorzustellen, welches über die reine Auswertung der Rohdaten hinaus hin zu einer Gesamtauswertung der Messdaten führt, welche für den Benutzer einen Mehrwert darstellt und über Zustände des Körpers informiert. Hierbei ist aufgrund der Sensibilität der Auswertung der hierfür erforderlichen Signale ein hohes Maß an Verlässlichkeit derselben erforderlich, so dass diesbezüglich eine Messvorrichtung mit einem besonders hohen Grad an Messgenauigkeit, insbesondere einen besonders hohen Grad an Erkennungswahrscheinlichkeit für Artefakte, erforderlich ist, welche in diesem Zusammenhang ebenfalls zu schaffen war.

Gelöst wird diese Aufgabe durch ein Verfahren zur Erfassung von Vitalparametern mithilfe einer Bioimpedanz-Messvorrichtung gemäß den Merkmalen des Anspruchs 1. Sinnvolle Ausgestaltungen des Verfahrens können den Unteransprüchen entnommen werden.

Im Rahmen der Betrachtung der vorliegenden Erfindung soll zunächst auf das Verfahren eingegangen werden, welches einerseits durchaus mit der ebenfalls anschließend beschriebenen Vorrichtung durchgeführt werden kann und vorzugsweise auch durchgeführt wird. Dennoch ist es möglich und soll in dem Schutzbereich mit enthalten sein, das fragliche Verfahren auch unabhängig von der hier beanspruchten Vorrichtung, also notfalls mit einer anderen Gerätekonfiguration, durchzuführen.

Erfindungsgemäß wird im Rahmen eines Verfahrens zur Erfassung von Vitalparametern eine Bioimpedanz-Messvorrichtung eingesetzt, bei der mithilfe eines ersten Elektrodensatzes ein Bioimpedanzsignal erfasst wird. Dieses Bioimpedanzsignal kann durch eine Elektrodenanordnung erfasst werden, welche zwei Einspeiseelektroden, welche in einem Abstand zueinander angeordnet sind, umfasst. Zwischen diesen beiden Einspeiselektroden sind, ebenfalls zueinander beanstandet, zwei Messelektroden angeordnet, welche das von den Einspeiseelektroden in die Haut eingespeiste Signal empfangen und zur Signalauswertung weiterleiten. Eine solche Anordnung aus zwei Einspeiseelektroden und zwei Messelektroden wird im Folgenden als ein "Elektrodensatz" bezeichnet. Mithilfe eines derartigen Elektrodensatzes wird also zunächst das Bioimpedanzsignal erfasst, demoduliert und dann einem Signalfilter zugeleitet. Mithilfe einer Tiefpassfilterung wird hierbei ein Atmungssignal isoliert, welches im Vergleich zum Blutpulssignal wesentlich langsamer oszilliert. Insoweit wird durch die Tiefpassfilterung die deutlich höhere Frequenz des Blutpulssignals ausgefiltert und die einander überlagerten Atmungs- und Blutpulssignale voneinander getrennt. Durch eine parallele Auswertung nach einer Hochpassfilterung wird zudem aus dem Bioimpedanzsignal das genannte Blutpulssignal abgeleitet, was eine erste Auswertungsstufe darstellt.

In einer zweiten Auswertungsstufe werden aus diesen beiden Signalen weitere abgeleitete Parameter bestimmt, welche beispielsweise die Atemfrequenz, die Herzrate sowie statistische und spektrale Parameter der Herzratenvariabilität umfassen. Diese Daten werden sodann, vorzugsweise innerhalb eines Mikrocontrollers, einer Auswertung zugeleitet, wobei der Mikrocontroller aus der Gesamtsituation, welche sich durch die genannten abgeleiteten Parameter darstellt, einen definierten Körperzustand ermittelt.

Derartige Körperzustände können im Wesentlichen Stresszustände, Wach-/Schlafzustände, insbesondere Schlafapnoen, sowie verschiedene Zustände des Herzkreislaufsystems umfassen. Auf diese Art und Weise ist es möglich, in einem mobilen, beispielsweise am Handgelenk tragbaren Langzeit-Messsystem Messungen zu diesen und weiteren Fragestellungen durchzuführen, und damit die Nutzungsschwelle für derartige Systeme herabzusetzen. Gleichzeitig ergeben sich für den Benutzer direkt auswertbare Informationen, für deren Auswertung aufgrund der vonseiten des Mikrocontrollers angestellten Zuordnung zu einem definierten Körperzustand, welcher beispielsweise mithilfe eines Klassifikators aus bekannten oder trainierten Zustandsbildern abgeleitet werden kann, kein weiteres Fachwissen und kein äußeres Zutun erforderlich ist. Eine zusätzliche, genauere Auswertung kann selbstverständlich durch den betreuenden Arzt ergänzt werden.

Um die Genauigkeit der Messung zu verbessern, ist wenigstens ein zweiter Elektrodensatz vorhanden, dessen Bioimpedanzsignal und gegebenenfalls direkt aus diesem nochmals abgeleitete Parameter, bedarfsweise allerdings auch nur das Bioimpedanzsignal, mit dem erstgenannten Signal verglichen wird. Hierdurch ist ein Auffinden von Artefakten ermöglicht, nachdem die Artefakte in Bezug auf die Pulsausbreitung an anderer Stelle auch versetzt auftreten, so dass diese aufgrund ihrer charakteristischen Form und ihrer versetzten Lage ermittelt und isoliert werden können.

Ein zweiter Elektrodensatz kann hierbei im selben Trägerband untergebracht sein, wie der erste Elektrodensatz, zusätzlich kann jedoch auch an einem zweiten Trägerband an anderer Stelle ein zusätzlicher Elektrodensatz vorhanden sein. Ergänzend sind auch mehr als zwei Elektrodensätze, welche in der genannten Art und Weise über den Körper verteilt sind, einsetzbar um das Messergebnis über die zusätzlich verfügbaren Signale weiter zu verbessern.

Eine noch weitere Verbesserung findet durch die Auswertung eines ebenfalls an dem Trägerband vorhandenen Bewegungssensors statt, bei dem es sich vorzugsweise um einen räumlichen Bewegungssensor handelt. Dieser erfasst die Bewegungssignale des Trägerbandes und damit zumindest einen Ausschnitt der Bewegungen des Benutzers. Es kann aufgrund dieser Bewegungssignale vonseiten des Mikrocontrollers ermittelt werden, in welcher Art und gegebenenfalls in welcher Frequenz Artefakte auftreten können, so dass bei einer starken Bewegung von einer Fortbewegung des Benutzers ausgegangen werden kann. Zudem können beispielsweise Erschütterungen, welche beim Gehen auftreten, auf diese Art und Weise lokalisiert und anschließend aus den Messdaten eliminiert werden. Im Falle zu starker Erschütterungen aufgrund der Bewegung des Nutzers kann aufgrund des Bewegungssignals auch vonseiten des Mikrocontrollers entschieden werden, dass einzelne Signale, oder auch alle Signale innerhalb eines Zeitintervalls, verworfen werden, wenn das Bewegungsmuster auf ein zu starkes Auftreten von Artefakten hinweist.

Neben diesem direkten Hinweis auf eine mögliche Störung des Messbetriebs werden sämtliche Signale, insbesondere Atmungssignal, Blutplussignal und die Bewegungssignale, aber auch die abgeleiteten Parameter, hinsichtlich ihrer Signalqualität ausgewertet, wobei insbesondere adaptive Filter und Tabellen mit Schwellwerten und Totzeiten verwendet werden können. Auch im Falle einer zu niedrigen Signalqualität kann der Mikrocontroller die Entscheidung treffen, Signale zu verwerfen oder auch Sensoren, welche eine zu niedrige Signalqualität, welche insbesondere unterhalb einer Qualitätsschwelle liegen, aufweisen, für ein Zeitintervall abzuschalten. Hierdurch können Sensoren, welche vernünftigerweise kein brauchbares Signal liefern, abgeschaltet werden, so dass eine Energieeinsparung bei derartigen Sensoren ermöglicht wird. Dadurch, dass eine Zuschaltung nach Ablauf des Zeitintervalls wieder vorgesehen ist, ist sichergestellt, dass die Messung nicht vollständig auf die möglicherweise nur zeitweilig nicht einsatzfähigen Sensoren verzichten muss.

In dem Fall, dass über einen längeren Zeitraum hinweg eine Auswertung der Signale nicht möglich ist, sei es weil die Signale unbrauchbar gewesen sind oder die Sensoren ganz abgeschaltet waren, so wird die Bioimpedanz-Messvorrichtung ein Signal in einer beliebigen Art und Weise, etwa ein taktiles, ein optisches und/oder ein akustisches Signal, aussenden, welches den Benutzer dazu auffordern soll, für die Dauer einer Zwischenmessung in einem Ruhezustand zu verharren, so dass eine allenfalls gewünschte Langzeitmessung auf genügend Stützpunkten über die Zeit hinweg beruht.

Im Rahmen des Verfahrens werden die Signalkurven sowohl des Atmungssignals als auch des Blutpulssignals, des Bewegungssignals und auch der Signale der abgeleiteten Parameter aufgezeichnet und innerhalb dieser Kurven nach charakteristischen Punkten und Strecken gesucht. Eine Auswertung dieser Kurven erfolgt im Rahmen der hier genannten Verfahren durch Auffinden der Maxima, Minima, der Fußpunkte, der Punkte maximaler Steigung und deren Abständen in Hoch- und Querrichtung, sowie der Flächen in der Hauptpulswelle und der reflektierten Pulswelle, sowie gegebenenfalls weitere Auffälligkeiten innerhalb der Signale, insbesondere um eine Aussage über den Zustand des Gefäßsystems und eine Schätzung von Blutdruckänderungen, soweit die genannten charakteristischen Punkte und Strecken das Blutpulssignal betreffend, anstellen zu können. Darüber hinaus ist es auch möglich, mithilfe eines zusätzlichen EKG-Signals und dessen entsprechender Auswertung eine Ermittlung der Pulstransitzeit zu leisten. Hierbei kann das EKG per Funk mit der Bioimpedanz-Messvorrichtung gekoppelt sein. Eine Ermittlung von Blutdruckänderungen kann in diesem Zusammenhang abgeschätzt werden.

Als weiterer Bestandteil der Sensorik der Bioimpedanz-Messvorrichtung kann ein Barometer vorhanden sein, aufgrund dessen Messwerten eine Berücksichtigung von Höhenänderungen für eine Verbesserung der Blutdruckschätzung ermöglicht wird.

Aus dem abgeleiteten Atmungssignal und den Daten der Pulswelle können ferner die Kohärenz zwischen Atmung und Herzaktivität bzw. die kardiorespiratorische Koordination bestimmt werden.

Eine Bestimmung der Stressbelastung kann aufgrund der Nutzung wenigstens eines der Parameter der Herzratenvariabilität aus dem Blutpuls, der kardiorespiratorischen Koordination, der Herzratenerhöhungen bei körperlicher Ruhe unter Verwendung der Signale des Blutpulses und des Bewegungssensors sowie aufgrund von Hautleitwertreaktionen, welche von einem Sensor für die elektrodermale Aktivität bestimmt werden, erfolgen.

Die Erkennung von körperlichen Aktivitäten erfolgt mithilfe einer Auswertung des Beschleunigungssensors und, soweit vorhanden, des zusätzlich vorzusehenden Barometers. Eine Beurteilung der körperlichen Fitness kann hierbei ergänzend aus dem Verlauf von Pulsfrequenz und Energieumsatz, welcher mithilfe des Beschleunigungssensors und gegebenenfalls des Barometers abgeschätzt werden kann, getroffen werden. Eine Verbesserung der Energieumsatzschätzung ist mithilfe der Pulsfrequenz und dem Beschleunigungssensor, sowie einem Drucksensor möglich. Bei statischen Aktivitäten erfolgt die Energieumsatzschätzung anhand der Pulsfrequenz.

Eine konkrete Umsetzung einer Bioimpedanz-Messvorrichtung sieht insbesondere einen ersten Elektrodensatz zur Kontaktierung der Haut des Benutzers vor, welcher nach dem Vier-Elektroden-Prinzip, wie eingangs erläutert, aus zwei außen liegenden Einspeiseelektroden und zwei innen liegenden, ebenfalls voneinander beabstandeten Messelektroden besteht. Auf einem Trägerband ist dieser erste Elektrodensatz so angeordnet, dass bei bestimmungsgemäßem Anlegen eine Abdeckung eines darunter liegenden Blutgefäßes, insbesondere der Arteria radialis, erfolgt, so dass das in der genannten Arterie pulsierende Blut die Impedanzänderungen, welche mithilfe der Sensorik erfasst werden, messbar beeinflusst. Eine Verbesserung der Messung erfolgt hierbei dadurch, dass ein zweiter Elektrodensatz beabstandet zu dem ersten Elektrodensatz derart ebenfalls dem Trägerband zugeordnet ist, dass die Elektroden des zweiten Elektrodensatzes den Bereich eines zweiten Blutgefäßes, idealerweise der Arteria ulnaris, zugeeignet wird. Dadurch, dass eine separate Messung für beide, parallel im Körper verlaufenden Blutgefäße ermöglicht ist, kann durch Vergleich der beiden in den jeweiligen Gefäßen ankommenden Signale bereits ein Ausscheiden einer Vielzahl von Artefakten vorgenommen werden. Auf einfache Weise wird daher die Signalqualität in der Vorrichtung deutlich verbessert.

Das Ausscheiden der Artefakte erfolgt hierbei dadurch, dass die beiden an den unterschiedlichen Gefäßen gemessenen Signale, welche zu unterschiedlichen Laufzeiten an dem Trägerband ankommen, verglichen werden. Nachdem Artefakte innerhalb des Körpers, welche beispielsweise auf der Bewegung des Körpers beruhen können, keine gleichartige Ausbreitung vom Herzen in Richtung des Trägerbandes erfahren, werden die Artefakte im Vergleich zum Puls an versetzten zeitlichen Stellen auftreten, so dass diese identifizierbar und dadurch auch eliminierbar sind.

Um das Messergebnis insoweit weiter zu verbessern, ist es vorgesehen, dass die Einspeiseelektroden flächenmäßig größer als die zugeordneten Messelektroden ausgeführt sind. Es wird angestrebt, das Verhältnis von Impedanzänderungen aufgrund des Blutpulses zur mittleren Impedanz zu maximieren und der Einfluss von Bewegungen auf die Impedanz zu minimieren. Insbesondere können derartige Elektroden aus einem leitfähigen Textil hergestellt sein, oder aus einem leitfähigen Kunststoff, vorzugsweise aus einem Polyurethan-Material gefertigt werden. Gleiches gilt für die die Elektroden innerhalb des Trägerbandes verbindenden elektrischen Leitungen. Das Trägerband als solches ist mit einigem Vorteil aus einem Textil gefertigt, welches sich an die Bewegungen und die Körperformen des Trägers anpasst. Hierdurch werden Handbewegungen des Benutzers von den Elektroden entkoppelt, so dass durch eventuelle Bewegungen im Bereich des Trägerbandes keine zusätzlichen Artefakte entstehen. Zudem ist durch ein textiles Trägerband der Tragekomfort erhöht. Eine Anbringung eines solchen Trägerbandes ist ohne Weiteres an verschiedenen Körperstellen, wie beispielsweise am Handgelenk, an den Fußgelenken, an der Stirn und an anderen für die Messung geeigneten Körperstellen im Rahmen der Erfindung möglich.

Zur Auswertung der Signale, welche der Mikrocontroller ermittelt und zumindest teilweise in einem Datenspeicher ablegt, kann eine Datenverbindung zu einem externen Datenverarbeitungsgerät, beispielsweise einem Smartphone, vorgesehen sein. Mithilfe eines derartigen externen Datenverarbeitungsgerätes kann eine zusätzliche Auswertung der Daten erfolgen, sowie eine zusätzliche Speicherung und Aufbereitung der Daten für den Nutzer. Eine Speicherung der Daten kann jedoch ebenfalls unabhängig hiervon innerhalb der Vorrichtung erfolgen.

Die vorstehend beschriebene Erfindung wird im Folgenden anhand eines Ausführungsbeispiels näher erläutert.

Es zeigen
- Figur 1: Eine schematische Darstellung des Trägerbandes und der darauf angeordneten Elektroden und Sensoren
- und Figur 2: das Trägerband gemäß Figur 1 in einem ausgebreiteten Zustand in einer Draufsicht von oben.

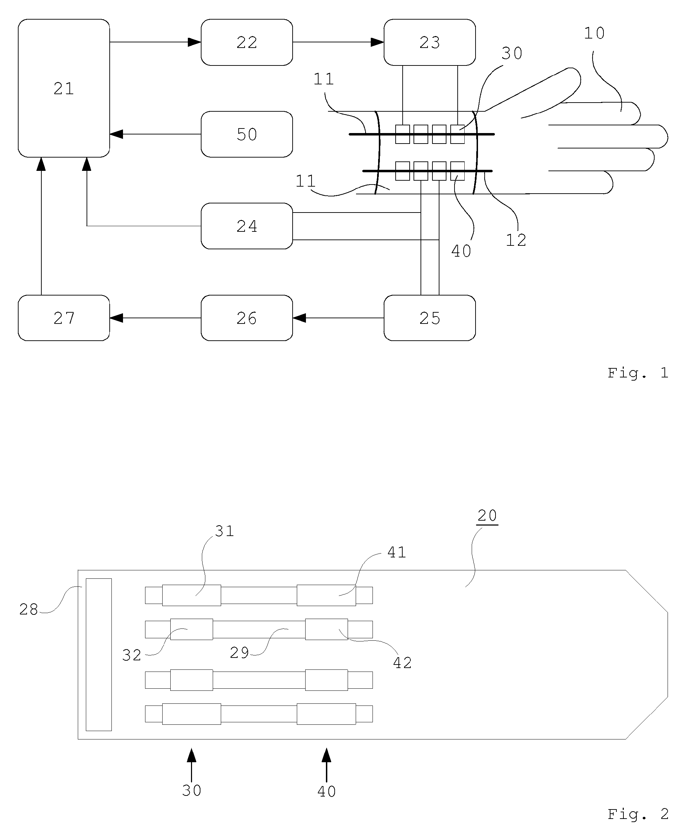

Figur 1 zeigt ein Beispiel für die Ausgestaltung eines Trägerbandes 20, welches für die Verwendung an dem Gelenk einer Hand 10 eines Benutzers ausgestaltet ist. In dem Trägerband 20 sind ein erster Elektrodensatz 30 und ein zweiter Elektrodensatz 40 angeordnet, wobei der erste Elektrodensatz 30 derart angeordnet ist, dass er bei bestimmungsgemäßer Anlage des Trägerbandes 20 über der Arteria radialis 11 zum Liegen kommt, während der zweite Elektrodensatz 40 im Bereich der Arteria ulnaris 12 angeordnet ist. Der erste Elektrodensatz 30 und auch der zweite Elektrodensatz 40 sind jeweils mit einer Stromversorgung 23 und einem Verstärker 25 verbunden, wobei in der Zeichnung nur jeweils die den Symbolen für Stromversorgung 23 und Verstärker 25 am nächsten liegenden Elektroden eine Verbindung aufweisen. Dies ist lediglich der zeichnerischen Übersicht geschuldet, tatsächlich sind jedoch die Messelektroden 32, 42 beider Elektrodensätze 30, 40 mit dem Verstärker 25, die Einspeiseelektroden 31, 41 beider Elektrodensätze 30, 40 mit der Stromversorgung 23 verbunden. Ebenfalls wird eine Gleichstrom- bzw. -spannungsmessung 24 bezüglich beider Messelektroden 32, 42 durchgeführt, dessen Messergebnisse an einen Mikrocontroller 21 zur Auswertung weitergeleitet werden. Der Mikrocontroller 21 steuert hierbei zunächst die Erzeugung eines sinusförmigen Stroms, welcher über eine Sinuserzeugung 22 erfolgt. Mithilfe des so erzeugten sinusförmigen Signals wird von der Stromversorgung 23 eine Versorgung der Einspeiseelektroden 31, 41 beider Elektrodensätze 30, 40 realisiert, wodurch nach dem bekannten Vier-Elektroden-Prinzip eine Bioimpedanz-Messung in jedem Elektrodensatz 30, 40 erfolgt. Die Erfassung eines Messsignals erfolgt über den Verstärker 25 und einen Demodulator 26, wobei anschließend eine Signalaufbereitung 27, im Wesentlichen eine Filterung vor einer A/D-Wandlung im Mikrocontroller 21, durchgeführt wird. Das so aufbereitete Signal wird in dem Mikrocontroller 21 weiter ausgewertet. Der Mikrocontroller 21 wird nach erfolgter Signalauswertung eine Verknüpfung der einzelnen Signale und daraus abgeleiteter Parameter vornehmen und so Rückschlüsse auf den Körperzustand des Benutzers ermöglichen und anstellen.

Figur 2 zeigt eine mögliche Ausgestaltung des Trägerbandes 20, welches die beiden Elektrodensätze 30 und 40 zeigt. Sowohl der erste Elektrodensatz 30, welcher über der Arteria radialis 11 zum Liegen kommen soll, als auch der zweite Elektrodensatz 40, welcher über der Arteria ulnaris 12 angeordnet werden soll, weisen jeweils zwei außen liegende Einspeiseelektroden 31, 41 auf, welche flächenmäßig größer ausgestaltet sind als die jeweils dazwischen liegenden Messelektroden 32, 42. Die den Elektroden unterliegenden textilen Leiter 29 befinden sich üblicherweise auf der Gegenseite des Trägerbandes 20 und sind hier nur der Vollständigkeit halber sichtbar mit angegeben. Die Verschaltung ist im Einzelnen hierbei als allgemein zu verstehen, auf eine Verschaltung wie hier angegeben ist nicht zu bestehen. Insbesondere ist es möglich, die beiden Elektrodensätze 30, 40 unabhängig voneinander auszuwerten, also die jeweiligen Elektroden nicht notwendigerweise kurz zu schließen. Mithilfe eines Verschlusses 28 wird das Trägerband 20 verschlossen und kann in geeigneter Art und Weise dem Benutzer am Handgelenk oder an anderer geeigneter Stelle zugeordnet werden.

Vorstehend beschrieben ist somit ein Verfahren zur Erfassung von Vitalparametern mithilfe einer Bioimpedanz-Messvorrichtung sowie eine hierfür geeignete Bioimpedanz-Messvorrichtung, wobei mithilfe dieses Verfahrens eine Ermittlung eines definierten Körperzustandes ermöglicht wird, welche über eine bloße Auswertung der Rohdaten zur Unterstützung des Benutzers deutlich hinausgeht. Die ebenfalls vorgeschlagene Bioimpedanz-Messvorrichtung sieht unterdessen Mittel vor, die Signalauswertung weiter deutlich zu verbessern, indem von vornherein mehrere Elektrodensätze in einem Trägerband untergebracht werden, welche parallel zur Auswertung kommen und so ein Herauslesen von Artefakten erleichtern.

## Patentansprüche

1. Verfahren zur Erfassung von Vitalparametern mithilfe einer am Handgelenk tragbaren Bioimpedanz-Messvorrichtung, wobei mithilfe wenigstens eines ersten Elektrodensatzes (30), bestehend aus einem Paar von Einspeiseelektroden (31) zur Einleitung eines Messstroms in die Haut eines Benutzers und einem zwischen den Einspeiseelektroden (31) voneinandei beabstandet angeordneten Paar von Messelektroden (32) zur Erfassung des Messstroms über die Haut, ein Bioimpedanzsignal erfasst wird, aus welchem ein Blutpulssignal und
durch Tiefpassfitterung ein Atmungssignal abgeleitet werden und das Atmungs- und das Blutpulssignal einer Signalauswertung zur Bestimmung abgeleiteter Parameter zugeführt werden,
wobei die abgeleiteten Parameter in einem Mikrocontroller ausgewertet werden, welcher aus der Gesamtheit der abgeleiteten Parameter einen definierten Körperzustand ermittelt,
**dadurch gekennzeichnet, dass** zur Auswertung der abgeleiteten Parameter wenigstens ein zweiter Elektrodensatz (40) vorgesehen ist, dessen Bioimpedanzsignal und dessen abgeleitete Parameter zum Auffinden von Artefakten mit dem Bioimpedanzsignal und dessen abgeleiteten Parametern des ersten Elektrodensatzes (30) verglichen werden und das Blutpulssignal durch Hochpassfilterung abgeleitet wird.

2. Verfahren gemäß Anspruch 1, **dadurch gekennzeichnet, dass** ergänzend zu der Bioimpedanzmessung mittels eines der Bioimpedanz-Messvorrichtung zugeordneten Bewegungssensors (50) Bewegungssignale erfasst und dem Mikrocontroller (21) zugeleitet werden.

3. Verfahren gemäß Anspruch 2, **dadurch gekennzeichnet, dass** mithilfe einer Auswertung der Bewegungssignale und der abgeleiteten Parameter Artefakte und/oder definierte Körperzustände bestimmt werden, wobei Artefakte entweder aus den Signalen eliminiert werden oder zu einem Verwerfen von einzelnen Signalen oder aller Signale in einem Zeitintervall führen.

4. Verfahren gemäß Anspruch 3, **dadurch gekennzeichnet, dass** der Mikrocontroller (21) Atmungssignal, Blutpulssignal und Bewegungssignale, sowie vorzugsweise auch abgeleitete Parameter, hinsichtlich der Signalqualität auswertet, wobei Sensoren mit einer Signalqualität unterhalb einer Qualitätsschwelle für ein Zeitintervall abgeschaltet werden.

5. Verfahren gemäß einem der Ansprüche 3 oder 4, **dadurch gekennzeichnet, dass** der Mikrocontroller die Bewegungssignale hinsichtlich der Bewegungsintensität auswertet und bei einer zu starken Bewegung die Sensoren und/oder die Signalauswertung für ein Zeitintervall abgeschaltet werden.

6. Verfahren gemäß einem der Ansprüche 4 oder 5, **dadurch gekennzeichnet, dass** im Falle unbrauchbarer Signale und/oder einer Abschaltung von Sensoren bzw. Signalauswertung über ein bestimmtes Zeitintervall hinweg von der Bioimpedanz-Messvorrichtung ein taktiles, optisches und/oder akustisches Signal ausgesandt wird.

7. Verfahren gemäß einem der vorhergehenden Ansprüche, **dadurch gekennzeichnet, dass** im Rahmen einer Signalauswertung des Blutpulssignals charakteristische Punkte und Strecken der Signalkurve ermittelt und ausgewertet werden.

8. Verfahren gemäß einem der vorhergehenden Ansprüche, **dadurch gekennzeichnet, dass** an definierten Körperzuständen zumindest ein Schlafzustand, insbesondere Schlafapnoen oder ein Stresszustand feststellbar ist.

## Claims

1. A method for detecting vital parameters by means of a bio-impedance measuring apparatus that can be worn on the wrist, wherein a bio-impedance signal is detected by means of a first set of electrodes (30), consisting of a pair of feed electrodes (31) for introducing a measurement current into the skin of a user and a pair of measurement electrodes (32) arranged spaced from each other between the feed electrodes (31) for detecting the measuring current via the skin, from which signal a blood pulse signal and, by low-pass filtering, a respiration signal are derived, and the respiration and blood pulse signal are supplied to signal evaluation for determining derived parameters, wherein the derived parameters are evaluated in a microcontroller which determines a defined physical condition from the totality of the derived parameters, **characterized in that** at least one second set of electrodes (40) is provided for evaluating the derived parameters, whose bio-impedance signal and whose derived parameters are compared with the bio-impedance signal and its derived parameters of the first electrode set (30) for finding artefacts, and the blood pulse signal is derived by high-pass filtering.

2. A method according to claim 1, **characterized in that** in addition to the bio-impedance measurement movement signals are detected and supplied to the microcontroller (21) by means of a movement sensor (50) assigned to the bio-impedance measuring apparatus.

3. A method according to claim 2, **characterized in that** artefacts and/or defined physical conditions are determined by means of an evaluation of the movement signals and the derived parameters, wherein the artefacts are either eliminated from the signals or lead to a rejection of individual signals or all signals in a time interval.

4. A method according to claim 3, **characterized in that** the microcontroller (21) evaluates the respiration signal, blood pulse signal and movement signals, and preferably also derived parameters, concerning the signal quality, wherein the sensors with a signal quality beneath the quality threshold are deactivated for a time interval.

5. A method according to one of the claims 3 or 4, **characterized in that** the microcontroller evaluates the movement signals concerning the movement intensity, and in the case of excessive movement the sensors and/or the signal evaluation are deactivated for a time interval.

6. A method according to one of the claims 4 or 5, **characterized in that** a tactile, optical and/or acoustic signal is emitted by the bio-impedance measuring apparatus in the event of unusable signals and/or a deactivation of sensors or signal evaluation over a specific time interval.

7. A method according to one of the preceding claims, **characterized in that** within the scope of signal evaluation of the blood pulse signal characteristic points and sections of the signal curve are determined and evaluated.

8. A method according to one of the preceding claims, **characterized in that** at least one sleep state, especially sleep apnea or a state of stress, can be determined in defined physical conditions.

## Revendications

1. Procédé pour l'acquisition de paramètres vitaux à l'aide d'un dispositif de mesure de la bio-impédance pouvant être porté au poignet, dans lequel un signal de bio-impédance est acquis à l'aide d'au moins un premier jeu d'électrodes (30) composé d'une paire d'électrodes d'alimentation (31) destinées à introduire un courant de mesure dans la peau d'un utilisateur et d'une paire d'électrodes de mesure (32) disposées entre les électrodes d'alimentation (31) à distance l'une de l'autre pour capter le courant de mesure passant par la peau, et un signal de pouls sanguin est dérivé de celui-ci, ainsi qu'un signal de respiration par filtrage passe-bas, et les signaux de respiration et de pouls sanguin sont transmis à une analyse des signaux afin de déterminer des paramètres dérivés, les paramètres dérivés étant analysés dans un microcontrôleur qui détermine, à partir de l'ensemble des paramètres dérivés, un état défini du corps, **caractérisé en ce qu'**en vue de l'analyse des paramètres dérivés, il est prévu au moins un deuxième jeu d'électrodes (40) dont le signal de bio-impédance et les paramètres qui en sont dérivés sont comparés avec le signal de bio-impédance du premier jeu d'électrodes (30) et les paramètres qui en sont dérivés afin de déceler des artefacts, et le signal de pouls sanguin est dérivé par filtrage passe-haut.

2. Procédé selon la revendication 1, **caractérisé en ce qu'**en complément de la mesure de la bio-impédance, des signaux de mouvement sont acquis au moyen d'un capteur de mouvement (50) associé au dispositif de mesure de la bio-impédance et transmis au microcontrôleur (21).

3. Procédé selon la revendication 2, **caractérisé en ce que** des artefacts et/ou des états définis du corps sont déterminés à l'aide d'une analyse des signaux de mouvement et des paramètres qui en sont dérivés, les artefacts étant soit éliminés des signaux, soit déterminant le rejet de certains signaux ou de tous les signaux dans un intervalle de temps.

4. Procédé selon la revendication 3, **caractérisé en ce que** le microcontrôleur (21) analyse la qualité du signal de respiration, du signal de pouls sanguin et des signaux de mouvement et de préférence aussi des paramètres dérivés, les capteurs dont la qualité de signal est inférieure à un seuil de qualité étant désactivés pendant un intervalle de temps.

5. Procédé selon l'une des revendications 3 ou 4, **caractérisé en ce que** le microcontrôleur analyse l'intensité de mouvement à partir des signaux de mouvement, et l'analyse des capteurs et/ou des signaux est désactivée pendant un intervalle de temps si les mouvements sont trop forts.

6. Procédé selon l'une des revendications 4 ou 5, **caractérisé en ce que** si des signaux sont inexploitables et/ou l'analyse des capteurs ou des signaux désactivée au-delà d'un certain intervalle de temps, le dispositif de mesure de la bio-impédance émet un signal tactile, visuel et/ou sonore.

7. Procédé selon l'une des revendications précédentes, **caractérisé en ce que** dans le cadre d'une analyse du signal de pouls sanguin, des points et des longueurs caractéristiques de la courbe de signal sont déterminés et analysés.

8. Procédé selon l'une des revendications précédentes, **caractérisé en ce qu'**au moins un état de sommeil, en particulier des apnées du sommeil, ou un état de stress peut être constaté parmi les états du corps définis.
